(19) Europäisches Patentamt
European Patent Office
Office européen des brevets

(11) Publication number: **0 140 710 A2**

# (12) EUROPEAN PATENT APPLICATION

(21) Application number: **84307481.6**

(22) Date of filing: **30.10.84**

(51) Int. Cl.⁴: **C 07 C 139/12**, C 07 C 143/525, C 11 D 1/12

(30) Priority: **01.11.83 GB 8329074**

(43) Date of publication of application: **08.05.85 Bulletin 85/19**

(84) Designated Contracting States: **AT BE CH DE FR IT LI NL SE**

(71) Applicant: **UNILEVER NV, Burgemeester s'Jacobplein 1 P.O. Box 760, NL-3000 DK Rotterdam (NL)**

(72) Inventor: **Baker, Clifford Keith, Yew Tree House Liverpool Road, Neston South Wirral Cheshire L64 7TW (GB)**
Inventor: **van Dijk, Pieter Mari, Lafontainepad 7, NL-2871 KR Schoonhoven (NL)**
Inventor: **Griffin, David Arthur, 12 Berwyn Avenue, Thingwall Wirral Merseyside L61 7UN (GB)**
Inventor: **Helmond, Johannes, Albrechtsveld 32, NL-2804 WC Gouda (NL)**
Inventor: **Rand, John Arthur, 16 Higher Bebington Road, Bebington Wirral Merseyside (GB)**
Inventor: **Stanley, Christopher Paul, 7 High Ash Mount Alwoodley, Leeds 17 West Yorkshire (GB)**
Inventor: **Stenton, Neil, 6 Churnway Greasby, Wirral Merseyside (GB)**
Inventor: **Tombs, Christopher, 9 Wells Close, Mickle Trafford Chester (GB)**

(74) Representative: **Fransella, Mary Evelyn et al, Unilever PLC Patents Division P.O. Box 68 Unilever House, London EC4P 4BQ (GB)**

(54) **Dialkyl sulphosuccinate composition and process for its preparation.**

(57) Dialkyl sulphosuccinates containing less than 0.3% maleate/fumarate may readily be prepared at high concentrations (77–90% by weight) by the sulphitation of the corresponding dialkyl maleates/fumarates in a mixture of $C_1$–$C_4$ alcohol and water, without the need for other solvents or special catalysts, because at this very high concentration level there exist coinciding regions of low viscosity at both reaction and storage temperatures. The sulphiting agent is used in solid form, and inorganic solids are removed by filtration or other physical separation methods at the end of the reaction.

EP 0 140 710 A2

# DIALKYL SULPHOSUCCINATE COMPOSITION AND PROCESS FOR ITS PREPARATION

The present invention relates to the preparation of salts of dialkyl esters of sulphosuccinates, at high concentrations. These materials are useful *inter alia* as detergent-active agents.

Dialkyl sulphosuccinates are materials of the general formula I:

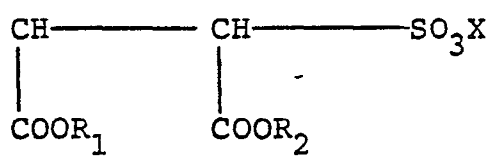

(I)

wherein $R_1$ and $R_2$ are straight-chain or branched-chain alkyl groups, and may be the same or different, and X is a monovalent cation or 1/m or *m*-valent cation. For detergent activity, the alkyl groups $R_1$ and $R_2$, which may be the same or different, generally have 4 to 12 carbon

atoms, and X is a solubilising cation, for example, alkali metal, ammonium, substituted ammonium or magnesium.

The synthesis of dialkyl sulphosuccinates is well documented in the literature, for example in US 2 028 091 and US 2 879 214 (American Cyanamid), EP 87 711A (Henkel), DD 75075 (Weidner), GB 1 576 019 (BASF), GB 1 215 561 (Melle-Bezons), GB 1 050 578 (Lankro) and GB 1 527 020 (Chemische Fabrik Pfersee). The preferred method for making these materials is by bisulphite addition to the corresponding dialkyl maleates and/or fumarates. The reaction is carried out in a generally aqueous medium, although various solvents may also be present, at temperatures above 85°C. The sulphiting agent is generally a water-soluble sulphite-ion-generating salt, for example, an alkali metal or ammonium sulphite, bisulphite or metabisulphite.

In order to achieve the highest possible conversion of maleate or fumarate to sulphosuccinate, the bisulphite or equivalent reagent is normally used in a molar excess. The product will then contain a certain amount of residual unreacted bisulphite. If the reducing action of this is objectionable, the product may be treated with an oxidising agent, for example, hydrogen peroxide, to convert any unreacted sulphite to sulphate. This involves an additional processing step, and does nothing to reduce the overall content of inorganic impurities in the product; it merely changes their chemical constitution. The amount of residual bisulphite or sulphite in the product may of course be reduced by using a smaller, or no, excess of this reagent, but either at the cost of less complete conversion from maleate/fumarate to sulphosuccinate within a reasonable reaction time, so that the product contains a relatively high level of starting material, or at the cost of a long reaction time. A long

reaction time, as well as being uneconomic and inconvenient, results in increased hydrolysis of both starting material and product to give a high level of the corresponding alcohols. Thus, in either case, although the amount of one impurity is lowered the amount of another is increased.

Another area of difficulty in the production of dialkyl sulphosuccinates concerns operation at high product concentrations, where intractably high viscosities at both reaction and storage temperatures can cause processing and handling problems. DD 75 075 (Weidner), GB 1 215 561 (Melle Bezons) and GB 1 527 020 (Chemische Fabrik Pfersee) do not disclose processes giving product concentrations higher then 70%.

GB 1 576 019 (BASF) describes a process for the production of sulphosuccinates in a wholly aqueous medium, without the use of solvents. Example 1 describes the production of di(2-ethylhexyl) sulphosuccinate at the high concentration of about 80% by weight. The product of this reaction is a solid gel, and is subsequently dissolved in a relatively large volume of isopropanol to give a product fluid at ambient temperature and containing about 68% active matter. The reactants (dialkyl maleate and aqueous sodium bisulphite solution) are used in stoichiometric proportions, although an excess of bisulphite is normally desirable, as explained previously, to drive the reaction to completion within a reasonable time; an excess is presumably avoided in the BASF example because it would be difficult to remove from the solidified product.

US 2 018 091 (American Cyanamid) describes, for example in Example 2, a similar preparation in water without solvents, at a product concentration of about 75%. An excess of sodium bisulphite (5 mole %) is used here,

but a special solvent extraction step with benzene is subsequently necessary to remove inorganic impurities from the product.

It has been proposed to carry out the reaction in a mixture of water and a lower alcohol, normally ethanol. This tends to lower the viscosity of the reaction mixture at any given temperature, but it also slows down the reaction, both because (at ambient pressure) the reaction temperature will be lower and because the concentration of sulphite species in solution will be lowered: sulphites and bisulphites are virtually insoluble in ethanol. The presence of ethanol does not prevent a steep rise in the viscosity at reaction temperature when the product concentration rises to about 60% by weight. This leads to long reaction times and significant product hydrolysis.

US 2 879 214 (American Cyanamid) discloses the use of ultraviolet light to promote and accelerate the preparation of dialkyl sulphosuccinates by sulphitation in an aqueous-alcoholic medium. The Examples were apparently carried out at product concentrations of 75% by weight, but despite the use of ultraviolet light relatively long reaction times of 4-5 hours were obtained, and in the absence of ultraviolet light the reaction time was as high as 7.33 hours, thus implying that the viscosity of the reaction mixture was relatively high. Despite the use of a stoichimetric excess of bisulphite, the content of unreacted maleate in these products was 0.45-0.7% by weight of the total reaction mixture, equivalent to 0.6-0.93% of the product itself; the content of inorganic impurities was about 1% by weight of the total, equivalent to 1.33% of the product.

EP 87 711A (Henkel) also relates to sulphosuccinate production in an aqueous-alcoholic medium, the reaction

being promoted by a free-radical catalyst. The processes of Example 2 and 4 to 7 are carried out at product concentrations of about 75% by weight, and are said to give products low in inorganic impurities, but excess maleate was used in each case so it is likely that the content of unreacted maleate in the products would be relatively high. Reaction times range from 2.5 to 6 hours when the free-radical catalyst is present, and it is to be presumed that these would be considerably longer in the absence of the catalyst.

According to GB 1 050 578 (Lankro) a non-polar water-immiscible solvent such as white spirit, kerosene or light mineral oil is included in the reaction medium. This is said to permit the preparation of homogeneous compositions containing from 40-75% by weight of dialkyl sulphosuccinate, although the highest concentration specifically disclosed in an Example is 62%.

GB 1 215 561 (Melle-Bezons) describes the production of dialkyl sulphosuccinates in aqueous-alcoholic solution in the presence of a small amount of dialkyl sulphosuccinate product from a previous batch. The relative proportions of water and alcohol are discussed, and it is stated that use of too high a proportion of alcohol results in solid bisulphite (sic) or metabisulphite remaining in the reaction medium throughout the reaction. This is presented as disadvantageous: the Melle-Bezons process is carried out in such a way that a homogeneous reaction mixture is always maintained. The final reaction mixture may be filtered if need be.

We have now discovered a method by means of which dialkyl sulphosuccinates may be prepared at high concentrations, with reaction times of 3 to 4 hours or less, using no solvents other than a lower alcohol and

water, to give a product liquid at both reaction and storage temperatures. The dialkyl sulphosuccinates obtained by our new process have low contents of unreacted maleate/fumarate in solution. Inorganic impurities can also be reduced to an extremely low level, and no oxidation step is required.

The product of our process is a composition having a viscosity of less than 10 poise, preferably less then 5 poise, at 40°C and $10s^{-1}$, and consisting essentially of from 77 to 90% by weight of $C_4$-$C_{16}$ dialkyl sulphosuccinate, from 1.5 to 15% by weight of a $C_1$-$C_4$ alcohol and from 5 to 20% by weight of water, the content of maleate/fumarate being less than 0.3% by weight, preferably less than 0.1% by weight, based on the dialkyl sulphosuccinate. The total content of inorganic material is preferably less than 1% by weight, more preferably less than 0.5% by weight, and the sulphite content preferably less than 0.5% by weight, more preferably less than 0.3% by weight, based on the dialkyl sulphosuccinate. The dialkyl sulphosuccinate preferably constitutes from 77 to 85% by weight of the composition.

The process of the invention is based on the discovery that dialkyl sulphosuccinates can be prepared in an aqueous-alcoholic medium at very high concentrations of from 77 to 90% by weight, because at that concentration level regions of unexpectedly low viscosity at reaction temperatures and at storage temperatures coincide. The sulphitation reaction will thus proceed rapidly and efficiently to completion within a reasonably short reaction time, and the product on cooling is easy to handle and store. At these high concentrations, solid inorganic material is present throughout the reaction, and can be removed at the end of the reaction by a physical separation method. If the ratio of alcohol to water is

relatively high and if the pH is above a certain value, a very high proportion of the inorganic material present is in solid form and will be removed in this way, so that a product low in inorganic impurities can be obtained.

Our discovery of an unexpected region of workably low viscosity at very high concentration levels has now enabled a physical separation method of removing inorganic impurities to be used, so that a comfortable excess of bisulphite, sufficient to ensure a very high conversion of maleate/fumarate to sulphosuccinate within a short reaction time, can be tolerated without giving a rise to a high level of inorganic matter in the product. The low viscosity at reaction temperature means that the reaction mixture can be stirred efficiently so that maximum contact between the reactants occurs and the reaction proceeds to completion within a reasonably short time, while the low viscosity at storage temperature means that the resulting product can be handled without difficulty.

The process of the invention accordingly comprises the steps of:

i) reacting together a water-soluble sulphite-ion-generating reagent in solid form and $C_4$-$C_{16}$ dialkyl maleate and/or fumarate, in a mole ratio of at least 1:1, the concentration of $C_4$-$C_{16}$ dialkyl maleate and/or fumarate being chosen so as to give a product concentration of from 77 to 90% by weight, the reaction being carried out in a mixture of a $C_1$-$C_4$ alcohol and water substantially free of other solvents, the reaction mixture comprising a liquid phase and a solid phase comprising inorganic material; and

ii) separating said liquid and solid phases, whereby there is obtained a composition (the liquid phase) comprising from 77 to 90% by weight of $C_4$-$C_{16}$ dialkyl sulphosuccinate having a dialkyl maleate and fumarate content of less than 0.3% by weight based on the dialkyl sulphosuccinate, from 1.5 to 15% by weight of $C_1$-$C_4$ alcohol and from 5 to 20% by weight of water, the composition remaining liquid down to a temperature of 40°C.

The concentration of dialkyl maleate/fumarate in step (i) is preferably chosen so as to give a product (dialkyl sulphosuccinate) concentration of from 77 to 85% by weight. Operation of the process at about 80% by weight product concentration appears to be optimal in terms of the rheology of both the reaction mixture and the subsequent cooled product.

The sulphiting reagent may be, for example, an alkali metal or ammonium sulphite or metabisulphite: sodium metabisulphite, which in water yields bisulphite ions, is a convenient and readily available choice. At the high concentration levels concerned, it will be necessary for the sulphiting reagent to be added to the reaction mixture in solid form: a saturated solution of sodium bisulphite, for example, contains only 35% by weight of bisulphite. Continuous mixing is required to aid dissolution of the solid sulphiting reagent as the reaction progresses.

The sulphiting reagent is preferably used in a greater than equimolar proportion in order to drive the reaction to completion in as short as possible a time, and is never used in less than equimolar proportions. A large molar excess is not, however, necessary: it is merely

necessary that solid inorganic material should be present. A molar excess of from 2 to 10%, preferably from 3 to 8%, has been found sufficient; higher levels merely mean larger amounts of solid in the reaction mixture.

The $C_1$-$C_4$ aliphatic alcohol may be, for example, methanol, isopropanol, isobutanol or glycerol, but ethanol is generally preferred for reasons of cost and convenience. This may be used in the form of industrial methylated spirits, which generally contain about 91 to 97.5% by weight of ethanol and about 0.5 to 4% by weight of methanol, the balance being water.

The ratio of $C_1$-$C_4$ alcohol to water in the reaction mixture at the end of the reaction is preferably at least 0.3:1, more preferably at least 0.5:1 and desirably at least 0.7:1.

If desired, the reaction may be carried out <u>ab initio</u> in a reaction mixture relatively rich in alcohol. Alternatively, the reaction may be carried out initially at a slightly higher concentration than that finally desired, in a mixture having a higher proportion of water. This gives increased bisulphite solubility and hence a faster reaction. Alcohol can subsequently be added to decrease the solubility of inorganic materials in the reaction mixture and thus to aid in their subsequent removal.

The levels of alcohol and water present in the reaction mixture are determined by various factors. At least 3% by weight of water, based on the dialkyl sulphosuccinate, is required for water of crystallisation of that product; at a product concentration of 80% by weight this represents a requirement of about 4% by weight on the total reaction mixture. If the solid sulphiting

agent is a metabisulphite, it will require a further 1.5% by weight of water, based on the total mixture at this concentration level, for its reaction to form bisulphite ion. Thus at 80% product concentration, at least about 5.5% by weight of water will be required, leaving room for at most 14.5% by weight of alcohol. In practice more water than this minimum quantity is usually desirable in order to provide sufficient sulphite species in solution to give a reasonable rate of reaction.

At least about 2% by weight of alcohol, based on the dialkyl sulphosuccinate, has been found necessary in order to enter the low viscosity region at reaction temperatures; at 80% product concentration this is of course equivalent to about 1.5% by weight of the total reaction mixture. In order to enter the low viscosity region at storage temperatures, however, about 6% by weight on dialkyl sulphosuccinate, equivalent to about 4.5% by weight of the total reaction mixture at 80% product concentration, is desirable. Furthermore, the higher the alcohol level, the more efficient will be the solids removal step which is an important feature of the process of the invention.

Preferred ranges are 10 to 15% by weight of water and 2 to 10% by weight of alcohol.

The reaction is normally carried out at boiling point at atmospheric or slightly elevated pressure. The actual temperature range will thus depend on the alcohol chosen and the proportions of alcohol to water used. If ethanol is the alcohol of choice, the reaction will generally be carried out at a temperature within the range of from 90 to 120°C.

Typical reaction times for the process of the invention range from 2 to 4 hours.

The process of the invention is operated within the low viscosity region unexpectedly discovered by the present inventors. The exact boundaries in concentration terms will depend on the proportions of alcohol and water present. To a much lesser extent they will also depend on the chain lengths of the dialkyl sulphosuccinates concerned.

Determinations of the viscosities of various dialkyl sulphosuccinate compositions in ethanol and water have shown that at 60% active matter the viscosities at temperatures of 20 to 80°C are all above 10 poise at high shear (110 $s^{-1}$) and in the 5-10 poise range at low shear (10 $s^{-1}$). At 75% active matter, viscosities at 30-40°C are well above 10 poise at both high and low shear, but drop to about 1 poise at 80°C (low shear) or 50°C (high shear): the composition is highly non-Newtonian. At 80% active matter, on the other hand, the viscosities at low and high shear differ very little, and are below 2 poise at 40°C and below 1 poise at 80°C. This virtually Newtonian behaviour greatly facilitates handling at both reaction and storage temperatures.

The relatively low viscosity at lower temperatures also facilitates the important step of physically separating inorganic solids at the end of the reaction: this is very difficult at viscosities above about 10 poise. The solid removal step may be carried out by any suitable method, filtration being preferred. Alternatively, the supernatant liquid, which contains the desired sulphosuccinate product, may be decanted. If desired, the solid phase may be recycled into subsequent processing batches.

The separation step will be more efficient, the less water-soluble the inorganic solid present. Since in

general sulphites are less soluble than bisulphites, it is therefore desirable that prior to the removal of inorganic solid, as much as possible of that solid should be in the form of sulphite rather than bisulphite. This requires a pH at the end of the reaction of at least about 7.

One way of ensuring that the residual solid is in sulphite form is by using sulphite as the sulphiting agent, but bisulphite is a more efficient sulphiting agent and the reaction proceeds faster at a pH of 5-6. If desired, the sulphiting agent may therefore consist of a mixture of (meta) bisulphite and sulphite. According to a preferred embodiment of the invention, however, the sulphiting agent consists wholly of (meta) bisulphite and there is also added to the reaction mixture a strongly alkaline reagent, in an amount sufficient to give a final pH within the range of from 6.0 to 8.5, preferably 6.5 to 7.5, the amount not exceeding that required to convert the whole excess of (meta) bisulphite present to sulphite. The amount added is preferably sufficient to convert approximately half of the excess of (meta) bisulphite used.

The strongly alkaline reagent is preferably sodium hydroxide, and my conveniently be added in the form of a concentrated aqueous solution, the water included being takein into account when calculating the total water requirement of the reaction mixture. The 47% aqueous caustic soda solution supplied commercially by ICI, Runcorn, England, is suitable.

Other strongly alkaline reagents, such as potassium hydroxide or gaseous ammonia, may be used instead.

The alkaline reagent is preferably present throughout the reaction, even though it can result in some reduction

in the rate of reaction. In principle its addition could be postponed until just before the solids removal step, but that procedure would involve a greater risk of product hydrolysis owing to the occurrence of regions of high local pH, so it is not preferred.

When an alkaline reagent is used to increase the efficiency of the solids removal step, the sulphite content of the product may be brought below 0.5%, and the levels of inorganic impurities in the product may be as low as 0.3% sulphite and 0.7% sulphate.

Control of the reaction pH by means of added hydroxyl ions is disclosed in DD 75 075 (Weidner) and GB 1 576 019 (BASF), both mentioned previously, but neither document discloses the separation of inorganic solids.

The process of the invention is of especial interest for preparing dialkyl sulphosuccinates having $C_6$-$C_8$ alkyl groups, which are highly efficient active ingredients for high-foaming liquid detergents. It may be used, for example, for preparing mixtures of dialkyl sulphosuccinates as described in GB 2 108 520A and GB 2 133 793A (Unilever).

Of especial interest is the preparation of $C_6/C_8$, $C_7/C_8$ and $C_6/C_7/C_8$ sulphosuccinate mixes from the reaction products of maleic anhydride with appropriate mixtures of $C_8$ and $C_6$ and/or $C_7$ aliphatic alcohols. These mixtures may be prepared at high concentrations by the process of the invention, and the resulting products of low maleate/fumarate and inorganic content may successfully be used to formulate a wide range of detergent products.

The following Examples illustrate the invention.

## EXAMPLE 1

A $C_6/C_8$ dialkyl maleate mixture was prepared from maleic anhydride and equimolar amounts of n-hexanol and n-octanol as described in Example 1 of GB 2 108 520 (Unilever).

The following reactants were placed in a 1.0 litre glass vessel.

| | |
|---|---|
| 324 g | $C_6/C_8$ dialkyl maleate |
| 42 g | ethanol (as 43 g of industrial methylated spirits) |
| 57 g | water |
| 62 g | 80% $C_6/C_8$ dialkyl sulphosuccinate composition (product of an earlier batch) containing 8% ethanol and 12% water |

The initial ethanol to water ratio was 0.73:1.

114 g of solid sodium metabisulphite (purity 95%), representing a 10% molar excess, were then added and the contents of the vessel sirred well. The mixture was heated to reflux and maintained at 96-100°C with stirring; the viscosity of the reaction mixture was less than 10 poise and stirring presented no difficulty. The endpoint of the reaction was determined as follows. A first crude indication of loss of sulphite ion was made using Merckoquant (Trade Mark) test papers, then the dialkyl maleate content of a sample was measured accurately using high performance liquid chromatography. After 2.5 hours, the reaction was judged to be complete; the stirrer was stopped and the mixture cooled slowly, allowing undissolved sodium sulphite/metabisulphite to settle out. The bottom layer of the vessel contents was removed, and the remainder, being the desired product, was allowed to cool to about 40°C, at which it was still a mobile liquid. Its dialkyl sulphosuccinate content was 82.60% by weight.

The dialkyl maleate content based on the dialkyl sulphosuccinate was 0.024%, and the final ethanol to water ratio was 0.87:1.

## EXAMPLE 2

The procedure of Example 1 was repeated using the following reactants:

| | |
|---|---|
| 324 g | $C_6/C_8$ dialkyl maleate |
| 42 g | ethanol (as 43 g of industrial methylated spirits) |
| 61 g | water |
| 58 g | 83% dialkyl sulphosuccinate composition (product of an earlier batch) containing 5% ethanol and 12% water |
| 114 g | sodium metabisulphite (95% pure) |

The initial ethanol to water ratio was 0.78:1. The viscosity of the reaction mixture was again below 10 poise, and the reaction was complete in 2 hours 40 minutes. The product, which on cooling to 40°C remained in the form of a mobile liquid, contained 82.40% by weight of dialkyl sulphosuccinate having a dialkyl maleate content of 0.024%. The final ethanol to water ratio was 0.82:1.

EXAMPLE 3

To an agitated batch reactor were charged:

264 parts of the $C_6/C_8$ dialkyl maleate mixture used in Example 1

15.2 parts ethanol (as 16 parts of industrial methylated spirits)

61 parts water

91 parts of solid sodium metabisulphite (95% pure)

48 parts of a 83% dialkyl sulphosuccinate composition (product of an earlier batch) containing 5% ethanol and 12% water.

The initial ethanol to water ratio was 0.26:1.

The reactor was heated to reflux and maintained at temperatures of 88-94°C, and pressures of 0 to 5 psig, for 4.25 hours. The reaction mixture had a viscosity of less than 10 poise and the agitator torque was satisfactorily low. After reaction, the mixture was cooled to 79°C and a further 20 parts of industrial methylated spirits (19

parts of ethanol) were added in order to aid separation of the residual sodium sulphite/metabisulphite. The ethanol to water ratio at this point was 0.63:1. After cooling further to 65°C, the agitator was stopped, and the inorganic precipitate allowed to settle before running off the bottom 20% of the reactor contents.

The remainder, being the desired product, was allowed to cool and remained as a mobile liquid at 40°C. It contained 80.00% by weight of dialkyl sulphosuccinate having a dialkyl maleate content of 0.18% by weight. Its final ethanol to water ratio was 0.63:1.

## EXAMPLE 4

This Example shows the use of a strongly alkaline reagent (sodium hydroxide solution) to give a product low in inorganic impurities.

To an agitated batch reactor were charged the following:

53.0 parts of the $C_6/C_8$ dialkyl maleate mixture used in previous Examples

11.7 parts water

7.0 parts industrial methylated spirits

0.4 parts 48% aqueous sodium hydroxide solution

16.8 parts sodium metabisulphite (solid, about 95% pure)

10.5 parts 79.6% dialkyl sulphosuccinate composition (product of an earlier batch) containing 8% ethanol and 11.4% water.

The reactor was heated to reflux and maintained at a temperature of 90-98°C at ambient pressure. The reaction mixture had a viscosity of less than 10 poise and the agitator torque was satisfactorily low. The reaction was complete in 4 hours. After the inorganic solids had been removed by filtration, the liquid phase was allowed to cool to 40°C, at which temperature it was still a mobile liquid. This product contained 82.00% dialkyl sulphosuccinate containing 0.07% dialkyl maleate and less than 0.4% inorganic impurities.

EXAMPLE 5

The procedure of Example 4 was repeated using dialkyl maleate derived from a mixture of 40 mole % n-hexanol and 60 mole % n-octanol. The materials charged to the batch reactor were as follows:

54.0 parts dialkyl maleate
11.9 parts water
7.2 parts industrial methylated spirits
0.4 parts 48% aqueous sodium hydroxide solution
17.0 parts sodium metabisulphite (solid, about 95% pure)
10.5 parts dialkyl sulphosuccinate composition (product of an earlier batch) containing 8% ehtanol and 12% water

The product contained 80.00% by weight of dialkyl sulphosuccinate having the exceptionally low dialkyl maleate content of 0.01% and an inorganic content of 0.5% (0.2% sulphite, 0.3% sulphate).

EXAMPLE 6

This Example show the viscosities at various temperatures of various dialkyl

sulphosuccinate/ethanol/water mixtures, measured by means of a Haake viscometer. Measurements above 80°C were not possible because the Haake viscometer cannot be used above that temperature.

The dialkyl sulphosuccinate used was the $C_6/C_8$ mixture, derived from 50 mole % n-hexanol and 50 mole % n-octanol, used in Examples 1 to 4. Each composition contained the stated percentage of dialkyl sulphosuccinate, 10% industrial methylated spirits, and water to 100%.

Since non-Newtonian behaviour was expected, viscosities were determined both at a low shear rate (10 $s^{-1}$) similar to that prevailing when pumping a composition to or from a storage tank, and a higher rate (100 or 110 $s^{-1}$) similar to that employed in stirring a reaction mixture. The results are shown in the Table.

| Active matter (%) | Shear rate ($s^{-1}$) | Viscosity (poise) at 30°C | 40°C | 50°C | 60°C | 70°C | 80°C |
|---|---|---|---|---|---|---|---|
| 60 | 10 | 40 | 45 | 45 | 40 | 30 | 25 |
| | 110 | 6 | 6 | 7 | 7 | 7 | 6 |
| 75 | 10 | 130 | 40 | 2.5 | 1.5 | 1.5 | <1 |
| | 110 | >20 | 12 | 1 | 1 | 1 | <1 |
| 80 | 10 | waxy | 1.7 | 1.2 | 0.65 | 0.6 | 0.5 |
| | 100 | solid | 1.3 | 1 | 0.6 | 0.5 | 0.3 |

The strongly non-Newtonian behaviour of the 75% composition and its relatively high viscosities at 40°C will be noted. The 80% composition, on the other hand,

displayed virtually Newtonian characteristics and all viscosities were well below 10 poise.

COMPARATIVE EXAMPLE A

An attempt was made to repeat Example 5 of EP 87 711A (Henkel), but without using a free-radical catalyst. Instead of di(2-ethylhexyl) maleate, an equivalent amount of the $C_6/C_8$ dialkyl maleate of Example 5 above was used.

In a 300 ml vessel fitted with a stirrer and a reflux condenser there was prepared a mixture of 53.3 g sodium metabisulphite (0.28 moles), 63.3 g water, 174.9 g (0.55 moles) of dialkyl maleate, and 28.6 g ethanol. The mixture was heated to 85°C and maintained at that temperature for 3.5 hours with vigorous stirring. The reaction mixture was initially in the form of two liquid phases and a solid phase, and after 3.5 hours no change was observed in its appearance. It was evident that very little conversion to dialkyl sulphosuccinate had occurred.

COMPARATIVE EXAMPLE B

Example 1 of GB 1 576 019 (BASF) was repeated using the $C_6/C_8$ dialkyl maleate of Examples 1 to 4 above instead of di(2-ethylhexyl) maleate.

143.1 g (0.45 moles) of dialkyl maleate were introduced into a vessel fitted with a stirrer, 47.5 g (0.45 moles) of sodium bisulphite in 43.7 g water were added, and the pH was adjusted to 5.5 with about 5 g of 50% aqueous sodium hydroxide solution. The mixture, which was initially very mobile and consisted of two liquid phases and a solid phase, was heated to 100°C and stirred at that temperature. After 1 hour 40 minutes, the reaction mixture became highly viscous and stirring

virtually stopped. After 2 hours, a sample was found to contain 77.4% dialkyl sulphosuccinate having a residual maleate content of less than 0.01%, and after a further two hours the dialkyl sulphosuccinate content of another sample was found to be 78.9%, still with a dialkyl maleate content of less than 0.01%.

On cooling, the already highly viscous reaction mixture became completely solid.

Thus the method of GB 1 576 019, using a wholly aqueous reaction and medium, can be operated at high active matter concentrations to produce dialkyl sulphosuccinate of high purity, but the product is virtually a solid even at reaction temperatures, and solidifies completely at lower temperatures.

According to Example 1 of GB 1 576 019, the solid product is subsequently mixed with isopropanol, but this of course produces a more dilute composition (about 68% active matter). The product obtained as described above was diluted with 90 parts by volume of isopropanol as instructed in GB 1 576 019, but the isopropanol was added in for equal parts and the viscosities at both high and low shear, at 40°C, were determined at each stage using the Haake viscometer. The results were as follows:

| Active matter (%) | Viscosity (poise) at 40°C $10\ s^{-1}$ | $110\ s^{-1}$ |
|---|---|---|
| 79.4 | 224 | 57 |
| 76.0 | 166 | 39 |
| 72.9 | 6 | 3 |
| 70.0 | 1.6 | 1 |
| 67.5 | 5.3 | 1.9 |

It will be seen that the active matter level had to be brought down to 73% before a low-shear viscosity below 10 poise was reached, the figures at 76 and 79.4% being extremely high. Viscosities of the order of 1 poise could only be achieved by a reduction of the active matter level to 70%. Thus the process described in GB 1 576 019 does not provide a route to low-viscosity liquid compositions containing 77% or more dialkyl sulphosuccinate.

CLAIMS

1. A process for the preparation of a dialkyl sulphosuccinate composition suitable for use as a raw material for detergent compositions, which process is characterised by the steps of

i) reacting together a water-soluble sulphite-ion-generating reagent in solid form, and $C_4$-$C_{16}$ dialkyl maleate and/or fumarate, in a mole ratio of at least 1:1, the concentration of $C_4$-$C_{16}$ dialkyl maleate and/or fumarate being chosen so as to give a product concentration of from 77 to 90% by weight, the reaction being carried out in a mixture of a $C_1$-$C_4$ alcohol and water substantially free of other solvents, the reaction mixture comprising a liquid phase and a solid phase comprising inorganic material; and

ii) separating said liquid and solid phases, whereby there is obtained a liquid phase comprising from 77 to 90% by weight of $C_4$-$C_{16}$ dialkyl sulphosuccinate having a dialkyl maleate and fumarate content of less than 0.3% by weight based on the dialkyl sulphosuccinate, from 1.5 to 15% by weight of a $C_1$-$C_4$ alcohol and from 5 to 20% by weight of water, said liquid phase remaining as a mobile liquid down to a temperature of 40°C.

2. A process as claimed in claim 1, characterised in that the concentration of the reactants in step (i) is chosen so as to give a product concentration within the range of from 77 to 85% by weight.

3. A process as claimed in claim 1 or claim 2, characterised in that the mole ratio of the sulphite-ion-generating reagent to the $C_4$-$C_{16}$ dialkyl maleate and/or fumarate is greater then 1:1.

4. A process as claimed in claim 3, characterised in that the water-soluble sulphite-ion-generating reagent is used in a molar excess within the range of from 2 to 10%.

5 A process as claimed in claim 4, characterised in that the water-soluble sulphite-ion-generating reagent is used in a molar excess within the range of from 3 to 8%.

6. A process as claimed in any one of claims 1 to 5, characterised in that the final weight ratio of $C_1$-$C_4$ alcohol to water is at least 0.3:1.

7. A process as claimed in any one of claims 1 to 6, characterised in that the $C_1$-$C_4$ alcohol comprises ethanol.

8. A process as claimed in any one of claims 1 to 7, characterised in that the water-soluble sulphite-ion-generating reagent comprises alkali metal metabisulphite.

9. A process as claimed in claim 8, characterised in that there is added to the reaction mixture a strongly alkaline reagent in an amount less than that required to convert all the excess of bisulphite present to sulphite but sufficient to give a final reaction pH within the range of from 6.0 to 8.5.

10. A process as claimed in claim 9, characterised in that the strongly alkaline reagent is added in an amount sufficient to give a final reaction pH within the range of from 6.5 to 7.5.

11. A process as claimed in claim 9 or claim 10, characterised in that the strongly alkaline reagent is aqueous sodium hydroxide solution.

12. A process as claimed in any one of claims 1 to 11, characterised in that in step (ii) the solid phase is removed by filtration.

13. A process as claimed in any one of claims 1 to 11, characterised in that in step (ii) the liquid phase is removed by decantation.

14. A process as claimed in any one of claims 1 to 13, characterised in that the alkyl groups of the dialkyl sulphosuccinate each have from 4 to 10 carbon atoms.

15. A process as claimed in claim 14, characterised in that the alkyl groups of the dialkyl sulphosuccinate each have from 6 to 8 carbon atoms.

16. A dialkyl sulphosuccinate composition suitable for use as a raw material for detergent compositions, characterised in that it consists essentially of

a) from 77 to 90% by weight of one or more water-soluble salts of $C_4$-$C_{16}$ dialkyl esters of sulphosuccinic acid,

b) from 1.5 to 15% by weight of a $C_1$-$C_4$ aliphatic alcohol, and

c) from 5 to 20% by weight of water,

the composition containing less than 0.3% by weight of $C_4$-$C_{16}$ dialkyl maleates and fumarates, based on the dialkyl sulphosuccinate (a), and having a viscosity of less then 10 poise at 40°C and 10 $s^{-1}$.

17. A composition as claimed in claim 16, characterised in that it has a viscosity of less than 5 poise at 40°C and 10 $s^{-1}$ .

18. A composition as claimed in claim 16 or claim 17, characterised in that the content of $C_4$-$C_{16}$ dialkyl maleates and fumarates is less than 0.1% by weight, based on the dialkyl sulphosuccinate (a).

19. A composition as claimed in any one of claims 16 to 18, characterised in that the content of sulphite is less than 0.5% by weight, based on the dialkyl sulphosuccinate (a).